Europäisches Patentamt

European Patent Office

Office européen des brevets

(11) Publication number:
**0 083 828**
**B1**

(12) **EUROPEAN PATENT SPECIFICATION**

(45) Date of publication of patent specification: **09.04.86**

(21) Application number: **82300115.1**

(22) Date of filing: **11.01.82**

(51) Int. Cl.⁴: **C 03 C 10/04,** C 03 C 10/16, A 61 K 6/06

(54) Stain-resistant mica compositions and articles thereof in particular dental constructs.

(43) Date of publication of application:
**20.07.83 Bulletin 83/29**

(45) Publication of the grant of the patent:
**09.04.86 Bulletin 86/15**

(84) Designated Contracting States:
**AT BE CH DE FR GB IT LI LU NL SE**

(56) References cited:
**EP-A-0 022 655**
**US-A-3 839 055**

**JOURNAL OF THE AMERICAN CERAMIC SOCIETY, vol. 55, no. 9, September 1972, pages 446-449, D.G. GROSSMAN: "Machinable glass-ceramics based on tetrasilicic mica"**

(73) Proprietor: **Corning Glass Works**
**Houghton Park**
**Corning New York 14831 (US)**

(72) Inventor: **Grossman, David Gary**
**200 Wall Street**
**Corning New York (US)**

(74) Representative: **Smith, Sydney et al**
**Elkington and Fife High Holborn House 52/54**
**High Holborn**
**London WC1V 6SH (GB)**

Courier Press, Leamington Spa, England.

## Description

The present invention relates to new materials useful for dental products and processes or the like, more specifically to new mica-based compositions for dental applications which exhibit improved resistance to staining in use.

A major purpose of the dental profession is to replace or correct damaged or deformed tooth structure by fabricating and installing dental constructs. Among the dental constructs frequently employed are dental appliances, e.g., denture plates, bridges or orthodontic brackets, attachments for such appliances, and dental prosthetic devices, e.g., inlays, onlays, partial or full dentures, crowns or caps.

All such dental constructs should be inert in the oral environment, be able to resist the forces of mastication, be capable of assuming a desired anatomical configuration, and exhibit aesthetic qualities similar to those of natural teeth. Few presently available materials simultaneously satisfy all of these requirements.

Present dental constructs are customarily composed of metal alloys, porcelain, amalgam, acrylic polymers or combinations thereof. Metal alloys and amalgams are undesirable in situations where aesthetics are important because they sharply differ from teeth in appearance.

Porcelain and acrylic polymers are too weak or brittle to resist masticatory forces in many locations.

Composite constructs comprising, for example, a metallic substructure and a porcelain superstructure for appearance are extremely technique sensitive, and are too bulky in many situations. Thus prior dental constructs have offered at best a compromise among the various requirements.

A recent and promising development in the field of dental treatment relates the use of mica glass-ceramics for the fabrication of dental constructs, as described by C.H. Pamiejer et al. in "Physical Properties of a Castable Ceramic Dental Restorative Material". AADR Abstract 327 *J. Dent. Res., 59* (March 1980) page 474. Glass-ceramics are semi-crystalline materials produced by the controlled cyrstallization *in situ* of glasses through an appropriate heat treatment, such materials having been first described in U.S. Patent No. 2,920,971. Because glass-ceramics are predominantly crystalline (more than 50% crystalline and frequently more than 90% crystalline by volume), they exhibit physical properties more closely akin to those of the predominating crystal phase than to those of the parent or precursor glass from which they are made. Thus glass-ceramics may vary widely in properties depending upon the particular crystal phases present therein.

Among the recently developed glass-ceramic materials are the mica-containing glass-ceramics, first described in U.S. Patent No. 3,689,293. Mica-containing glass-ceramics demonstrate a relatively unique property which renders them particularly desirable for the fabrication of dental tools and constructs, in that they deviate from brittle behaviour in a manner permitting them to withstand point impact without fracture propagation. Thus mica glass-ceramic bodies can be indented in a point hardness test procedure wherein conventional porcelains are fractured. This behaviour is attributed to the fact that the mica crystals of the predominating crystal phase can flow plastically to some extent through transitional gliding along the basal or cleavage plane.

The glass-ceramics of U.S. Patent No. 3,689,293, have been referred to as machinable glass-ceramics because they can be cut and shaped using conventional metal-working tools and procedures. They comprise a predominant crystal phase denominated fluorphlogopite solid solution (e.g. comprising fluorphlogopite $(KMg_3ALSi_3O_{10}F_2)$ and/or boron fluorphlogopite $[KMg_3BSi_3O_{10}F_2]$ crystals). Notwithstanding the excellent machinability of the above-described materials. C.H. Pameijer et al. suggests in their aforementioned publication the use of tetrasilicic mica glass-ceramic compositions to produce dental restorative materials. This family of glass-ceramics, described in U.S. Patent No. 3,732,087, characteristically includes tetrasilicic fluormica (e.g. $KMg_{2.5}Si_4O_{10}F_2$) crystals as the predominant crystal phase. A further description of the use of these materials for dental applications is found in published European Patent Application 22,655.

Tetrasilicic fluormica glass-ceramic differ from glass-ceramics containing fluorphlogopite and boron fluorphlogopite fluormica solid solutions in that both $Al^{+3}$ and $B^{+3}$ are eliminated from the crystal structure in favor of an added silicon ($Si^{+4}$) constituent. By virtue of this replacement, tetrasilicic glass-ceramic materials exhibit somewhat superior chemical durability and mechanical strength, U.S. Patent Nos. 3,689,293 and 3,732,087 are expressly incorporated herein by reference and ·may be consulted for a complete description of these known types of mica glass-ceramic materials.

It has of course been recognized that these tetrasilicic fluormica glass-ceramic compositions can contain minor additions of constituents other than those essential for the development of the characteristic tetrasilicic fluormica crystal phase. Hence, in "Machineable Glass-Ceramics Based on Tetrasilicic Mica", D.G. Grossman, *Journal of the American Ceramic Society,* Vol. 55, No. 9 (September 1972) pages 446—449, compositions comprising additions of $As_2O_5$ and $ZrO_2$ which still develop tetrasilicic flourmica crystals on appropriate heat treatment, and also exhibit other properties analogous to glass-ceramics of this type, are described.

2

For dental construct applications, EP—A—22655 discloses that tetrasilicic fluormica glass-ceramic compositions may suitably be prepared from a glass batch having the following composition (expressed in weight percent on the oxide basis):

| | |
|---|---|
| SiO₂ | 45—70 |
| MgO | 8—20 |
| MgF₂ | 8—15 |
| R₂O + RO | 5—35 |
| R₂O | 5—25 |
| RO | 5—20 |

wherein $R_2O$ consists of at least one oxide selected in the indicated proportion from: 0—20% $K_2O$, 0—23% $Rb_2O$, 0—25% $Cs_2O$, and wherein RO consists of at least one oxide selected from SrO, BaO and CdO. The glass batch may additionally contain up to 5% of glass colourants. Among the colourants proposed are the known transition metal colourants Mn, Fe, etc., and also oxides such as cerium oxide, titanium oxide and zirconium oxide. The publication also notes that minor additions of $B_2O_3$ and/or $Al_2O_3$ are possible, the effect of such additions being the alteration of the growth habits of the tetrasilicic fluormica crystal phase, and that minor additions of other oxides, such as $P_2O_5$, $TiO_2$, $ZrO_2$, FeO, ZmO, $GeO_2$, MnO, $La_2O_3$ and $SnO_2$ are useful, for example, in controlling the properties of the parent glass and the residual glassy phase.

Summary of the Invention

The present invention is concerned with an improvement in tetrasilicic mica glass-ceramics for dental construct fabrication and other uses, wherein a considerable enhancement in stain resistance is obtained. Stain resistance is of prime importance for dental prostheses and the like, but is also desirable for applications such as culinary ware, wherein contact with staining media is anticipated to be frequent.

In accordance with the invention, thermally crystallizable glasses for tetrasilicic fluormica glass-ceramic exhibiting greatly enhanced stain resistance are provided by adding minor controlled amounts of $Al_2O_3$ and $ZrO_2$ to selected tetrasilicic fluormica compositions. Thus the invention comprises a thermally crystallizable glass and stain-resistant tetrasilicic fluormica article, such as a dental construct, produced by the crystallization *in situ* of that glass. The glass and glass-ceramic articles of the invention desirably have a composition consisting essentially, in weight percent as calculated from the batch, of about 45—70% $SiO_2$, 8—20% MgO, 8—15% $MgF_2$, 5—20% $K_2O$, 0.05—2% $Al_2O_3$, 0.5—7% $ZrO_2$, 1—9% total of $ZrO_2 + Al_2O_3$, 0—7% $TiO_2$, and 0—10% total of conventional glass colourants. Of course, these compositions may additionally comprise other constituents known to be compatible with tetrasilicic fluormica compositions, including but not being limited to many of the Group II metal oxides and oxides of the metallic transition elements.

Glasses within the above-described composition range may be crystallized *in situ* to glass-ceramic articles by a nucleation and crystallization heat treatment of the kind conventionally used for glass-ceramic manufacture. A suitable treatment comprises heating the glass to a nucleation temperature in the range of about 650—850°C for a time in the range of 0.25—10 hours, and to a crystallization temperature in the range of 800—1200°C for a time in the range of about 1—100 hours.

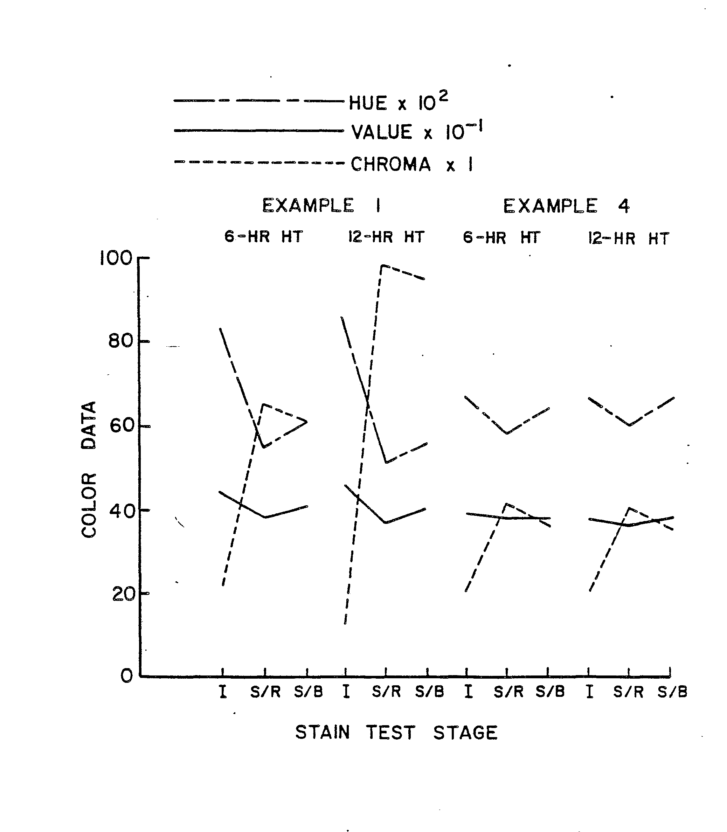

The invention may be further understood by reference to the single figure of the accompanying drawing which demonstrates the highly stain resistant character of the glass-ceramic material of Example 4 provided in accordance with the invention containing both $ZrO_2$ and $Al_2O_3$, compared with that of Example 1 containing only $ZrO_2$, as compared with that of Example 1.

An objective technique for evaluating the colour of teeth and candidate materials for dental constructs has recently been described by D.G. Grossman et al in "Evaluation of the Color of a Cast ceramic Restorative Material", AADR Abstract 1094, *J. Dent. Res., 59* (March 1980) page 542. That technique involves the colour analysis of light reflected from a material sample utilizing a photometer such as the Chromascan[R] shade scanner (commercially available from the Sterndent Corporation, Stamford, Conn.). In the operation of this scanner, the intensity of light reflected from a material to be evaluated is measured in red, green and blue regions of the spectrum and the resulting values are used to calculate the hue (predominant colour), chroma (colour saturation) and value (overall lightness on a white-to-black scale) of the reflected light. The stain resistance of a candidate material can be objectively measured by carrying out the colour evaluation before and after subjecting the material to a staining treatment.

Using the technique of the above-referenced Grossman et al publication it was found that, within the normal colouration range established in human teeth, hue ranged from about 0.2—0.8, chrome from about 20—180 and value from about 350—800. It is desirable in developing a dental restorative material to provide reflected colour characteristics within these limits, and this can ordinarily be accomplished by adding colourant to the material. What is more important is that the material does not exhibit large colour changes in use, hence the importance of developing a base material for construct fabrication exhibiting excellent stain resistance.

Coffee is one staining medium which is of particular concern in developing dental restorative materials, being categorized as one of the more severe staining media insofar as dental and/or culinary application are concerned. One staining test which is presently relied upon to evaluate and compare the stain resistance of candidate materials is a coffee stain test wherein a sample of a selected material is exposed to coffee for a seven-day exposure interval at 80°C. This test produces measurable changes in hue, value and chroma in many ceramic materials.

Compositions containing both $Al_2O_3$ and $ZrO_2$, in accordance with the invention show a better stain resistance than those which contain $ZrO_2$ only, as the Examples show.

The effect of small alumina and zirconia additions upon the stain resistance of glass-ceramic restorative materials as measured by the coffee stain test was demonstrated by comparing the performance of six candidate glass-ceramic materials in the test. The compositions of these materials are reported in Table I below, in parts by weight as calculated from the batch. The base constituents $SiO_2$, $K_2O$, MgO and $MgF_2$ total 100 parts so that their reported values correspond to weight percent of the base glass. The remaining constituents can be considered additions in excess of 100% to the base glass composition. It is noted that Examples 4—6 correspond, respectively, to Examples 1—3 except for the inclusion of a small amount of $Al_2O_3$ therein. Examples 1 to 3 are outside the scope of the invention but are retained for purposes of comparison.

TABLE I

|  | 1 | 2 | 3 | 4 | 5 | 6 |
|---|---|---|---|---|---|---|
| $SiO_2$ | 60.5 | 64.0 | 61.1 | 60.5 | 64.0 | 61.1 |
| $K_2O$ | 15.5 | 14.4 | 15.7 | 15.5 | 14.4 | 15.7 |
| $MgF_2$ | 10.5 | 9.7 | 10.4 | 10.5 | 9.7 | 10.4 |
| MgO | 13.5 | 11.9 | 12.8 | 13.5 | 11.9 | 12.8 |
| $ZrO_2$ | 2.0 | 2.0 | 1.0 | 2.0 | 2.0 | 1.0 |
| $TiO_2$ | — | — | 3.0 | — | — | 3.0 |
| $Al_2O_3$ | — | — | — | 0.5 | 0.5 | 0.5 |
| Colourants | — | — | 0.65 | — | — | 0.65 |

All of the materials reported in Table I are tetrasilicic fluormica glass-ceramic compositions, containing $SiO_2$, $K_2O$, $MgF_2$ and MgO as essential crystal-forming constituents and in some cases $TiO_2$ and colourants to control reflected colour. Glass-ceramics of these compositions were produced by forming molten glass of each specified composition into glass patties and converting the glass patties to highly crystalline glass-ceramics through heat treatment. Peak crystallization temperatures in the range of 1075—1090°C and crystallization times of 6 hours were utilized. Plates approximately 0.25 inches (0.64 cm) in thickness were cut from the glass-ceramic patties and polished prior to testing.

In conducting the stain test, each sample was first scanned for colour, then immersed in coffee at 80°C for seven days, thereafter removed, rinsed and deionized water and re-scanned for colour, and finally colour-scanned a third time after brushing for one minute with a commercially available dentifrice. The data from these tests are reported in Table II below. Included in Table II are hue (H), value (V), and chroma (C) results for each of the six samples, obtained prior to staining (initial) after staining and rinsing (stained/rinsed), and after brushing of the stained and rinsed samples (stained/brushed).

# 0 083 828

TABLE II

| Sample | Initial Colour | | | Stained/Rinsed Colour | | | Stained/Brushed Colour | | |
|---|---|---|---|---|---|---|---|---|---|
| No. | H | V | C | H | V | C | H | V | C |
| 1 | 0.83 | 440 | 22 | 0.55 | 380 | 65 | 0.60 | 400 | 61 · |
| 2 | 0.57 | 500 | 22 | 0.53 | 430 | 55 | 0.58 | 450 | 46 |
| 3 | 0.33 | 380 | 24 | 0.55 | 330 | 116 | 0.58 | 350 | 123 |
| 4 | 0.67 | 390 | 20 | 0.57 | 370 | 41 | 0.63 | 370 | 36 |
| 5 | 0.52 | 520 | 26 | 0.59 | 490 | 46 | 0.62 | 500 | 39 |
| 6 | 0.48 | 360 | 37 | 0.52 | 300 | 79 | 0.57 | 320 | 80 |

From a study of the data reported above in Table II, and of similar data recorded for glass-ceramic articles having the compositions of Table I but produced using alternative heat treatments and/or having different surface finishes, it appears that although the stain resistance of compositions 1—3 containing $ZrO_2$ alone would be acceptable in some cases alumina additions such as employed with compositions 4—6 above further improve stain-resistance regardless of the thermal history of the glass-ceramic material or surface finish thereof. Although, as suggested by the data in Table II, such additions appear to be most effective in reducing value and/or chroma shifts, hue stabilization is also observed in many cases.

The colour stabilizing effect of these additions is diagrammatically shown in the drawing, which presents selected colour data from Table II relating to compositions 1 and 4, and also additional coffee stain test colour data for these same two compositions, but after crystallization at peak temperature for 12 hours rather than 6 hours. The graph plots hue, value and chroma data on the vertical axis against stain test stage on the horizontal axis, the test stages shown being the initial (I), stained/rinsed (S/R) and stained/brushed (S/B) stages of the test. For examples 1 and 4 of Table I after crystallization *in situ* for six hours (6-hr. HT) and twelve hours (12-hr. HT).

The colourants which may be included in the compositions according to the invention include manganese oxide, iron oxide, nickel oxide or any of the other compounds or elements having known utility for colouring glass. The presence of zirconia in these compositions is particularly important not only to obtain enhanced acid durability, but also to control the translucency of the glass-ceramic product and to inhibit recrystallization in the glass-ceramic at high crystallization temperatures.

Although the mechanism by which coffee and other staining media impart permanent stains to tetrasilicic mica glass-ceramics is not well understood, it is believed that factors other than composition, including the thermal history of the glass-ceramic and the nature and extent of crystallization thereof, may also play some role in governing the ultimate stain resistance of these materials. Nevertheless, the effectiveness of small zirconia and alumina additions to enhance durability in a given composition, these other factors remaining constant is believed to constitute a significant improvement in tetrasilicic fluormica compositions for dental construct and other applications.

## Claims

1. A thermally crystallizable glass consisting essentially in weight percent as calculated from the batch, of about 45—70% $SiO_2$, 8—20% MgO, 8—15% $MgF_2$, 5—20% $K_2O$, 0.05—2% $Al_2O_3$, 0.5—7% $ZrO_2$, 1—9% total of $ZrO_2 + Al_2O_3$, 0—7% $TiO_2$, and 0—10% total of conventional glass colourants crystallizable to a tetrasilicic fluormica glass-ceramic material exhibiting excellent stain resistance.

2. A glass as claimed in claim 1 having a composition consisting essentially in weight percent calculated from the batch of 60.5% $SiO_2$, 15.5% $K_2O$, 10.5% $MgF_2$, 13.5% MgO, 2.0% $ZrO_2$ and 0.5% $Al_2O_3$.

3. A glass as claimed in claim 1 having a composition consisting essentially in weight percent calculated from the batch of 64.0% $SiO_2$, 14.4% $K_2O$, 9.7% $MgF_2$, 11.9% MgO, 2.0% $ZrO_2$ and 0.5% $Al_2O_3$.

4. A glass as claimed in claim 1 having a composition consisting essentially in weight percent calculated from the batch of 61.1% $SiO_2$, 15.7% $K_2O$, 10.4% $MgF_2$, 12.8% MgO, 1.0% $ZrO_2$, 3.0% $TiO_2$, 0.5% $Al_2O_3$ and 0.65% colourants.

5. A stain resistant tetrasilicic fluormica glass-ceramic article having a composition as specified in any of claims 1 to 4.

6. An article as claimed in claim 5 in the form of a dental construct.

5

**Patentansprüche**

1. Thermisch kristallisierbares Glas, bestehend im wesentlichen, in Gew.-%, berechnet aufgrund des Glasgemenges, aus etwa 45 bis 70 % $SiO_2$, 8 bis 20 % MgO, 8 bis 15 % $MgF_2$, 5 bis 20 % $K_2O$, 0,05 bis 2 % $Al_2O_3$, 0,5 bis 7 % $ZrO_2$, 1 bis 9 % insgesamt $ZrO_2 + Al_2O_3$, 0 bis 7 % $TiO_2$ und 0 bis 10 % insgesamt herkömmlicher Glasfärbungsmittel, kristallisierbar zu einem ausgezeichnete Verfärbungsbeständigkeit aufweisenden Tetrasilicat-Fluorglimmer-Glas-Keramikmaterial.

2. Glas nach Anspruch 1 mit einer Zusammensetzung bestehend im wesentlichen, in Gew.-%, berechnet aufgrund des Glasgemenges, aus 60,5 % $SiO_2$, 15,5 % $K_2O$, 10,5 % $MgF_2$, 13,5 % MgO, 2,0 % $ZrO_2$ und 0,5 % $Al_2O_3$.

3. Glas nach Anspruch 1 mit einer Zusammensetzung, bestehend im wesentlichen aus, in Gew.-%, berechnet aufgrund des Glasgemenges, 64,0 % $SiO_2$, 14,4 % $K_2O$, 9,7 % $MgF_2$, 11,9% MgO, 2,0 % $ZrO_2$ und 0,5 % $Al_2O_3$.

4. Glas nach Anspruch 1 mit einer Zusammensetzung, bestehend im wesentlichen aus, in Gew.-%, berechnet aufgrund des Glasgemenges, 61,1 % $SiO_2$, 15,7 % $K_2O$, 10,4 % $MgF_2$, 12,8 % MgO, 1,0 % $ZrO_2$, 3,0 % $TiO_2$, 0,5 % $Al_2O_3$ und 0,65 % Färbungsmittel.

5. Verfärbungsbeständiges Tetrasilicat-Fluorglimmer-Glas-Keramikerzeugnis mit einer Zusammensetzung, wie in irgend einem der Ansprüche 1 bis 4 angegeben.

6. Erzeugnis nach Anspruch 5 in Form einer Dentalkonstruktion.

**Revendications**

1. Verre thermiquement cristallisable formé essentiellement, en pourcentages pondéraux calculés à partir de la charge de départ, d'environ 45 à 70% de $SiO_2$, 8 à 20% de MgO, 8 à 15% de $MgF_2$, 5 à 20% de $K_2O$, 0,05 à 2% d'$Al_2O_3$, 0,5 à 7% de $ZrO_2$, 1 à 9% au total de $ZrO_2 + Al_2O_3$, 0 à 7% de $TiO_2$ et 0 à 10% au total de colorants classiques pour verres, cristallisables en un matériau vitrocéramique au fluormica tétrasilicique présentant une excellente résistance au tachage.

2. Verre selon la revendication 1, ayant une composition formée essentiellement, en pourcentages pondéraux calculés à partir de la charge de départ, de 60,5% de $SiO_2$, 15,5% de $K_2O$, 10,5% de $MgF_2$, 13,5% de MgO, 2,0% de $ZrO_2$ et 0,5% d'$Al_2O_3$.

3. Verre selon la revendication 1, ayant une composition formée essentiellement, en pourcentages pondéraux calculés à partir de la charge de départ, de 64,0% de $SiO_2$, 14,4% de $K_2O$, 9,7% de $MgF_2$, 11,9% de MgO, 2,0% de $ZrO_2$ et 0,5% d'$Al_2O_3$.

4. Verre selon la revendication 1, ayant une composition formée essentiellement, en pourcentages pondéraux calculés à partir de la charge de départ, de 61,1% de $SiO_2$, 15,7% de $K_2O$, 10,4% de $MgF_2$, 12,8% de MgO, 1,0% de $ZrO_2$, 3,0% de $TiO_2$, 0,5% d'$Al_2O_3$ et 0,65% de colorants.

5. Objet en vitrocéramique au fluormica tétrasilicique résistant au tachage ayant une composition telle que spécifiée dans l'une quelconque des revendications 1 à 4.

6. Objet selon la revendication 5 sous la forme d'une construction dentaire.